Europäisches Patentamt

**European Patent Office**

**Office européen des brevets**

(11) Veröffentlichungsnummer:

**0 035 185**
A1

(12) **EUROPÄISCHE PATENTANMELDUNG**

(21) Anmeldenummer: 81101203.8

(22) Anmeldetag: 20.02.81

(51) Int. Cl.³: **C 07 D 235/28**

(30) Priorität: 04.03.80 DE 3008159

(43) Veröffentlichungstag der Anmeldung: **09.09.81**
**Patentblatt 81/36**

(84) Benannte Vertragsstaaten: **BE DE FR GB IT**

(71) Anmelder: **BAYER AG, Zentralbereich Patente, Marken und Lizenzen, D-5090 Leverkusen 1, Bayerwerk (DE)**

(72) Erfinder: **Schubart, Rüdiger, Dr., An der Engelsfuhr 27, D-5060 Bergisch-Gladbach 2 (DE)**
Erfinder: **Langner, Günter Paul, Dr., Elisabethlaan 63, B-2600 Berchem (BE)**

(54) Verfahren zur Herstellung von 4- bzw. 5-Methyl-2- mercaptobenzimidazol.

㉗ Verfahren zur Herstellung von 4- bzw. 5-Methyl-2-mercaptobenzimidazol, dadurch gekennzeichnet, dass man 1 Gewichtsteil 4- bzw. 5-Methylbenzimidazolon mit 3 bis 10 Gewichtsteilen Schwefelkohlenstoff in Gegenwart von 1 bis 12 Gewichtsprozent eines basischen Katalysators, bezogen auf Benzimidazolon, in einem Temperaturbereich von 200 bis 300°C umsetzt.

EP 0 035 185 A1

ACTORUM AG

- 1 -

0035185

BAYER AKTIENGESELLSCHAFT       5090 Leverkusen, Bayerwerk

Zentralbereich
Patente, Marken und Lizenzen   E/Kü-c

Verfahren zur Herstellung von 4- bzw. 5-Methyl-2-
mercaptobenzimidazol

Die Erfindung betrifft ein Verfahren zur Herstellung
von 4- bzw. 5-Methyl-2-mercaptobenzimidazol durch
Umsetzung eines entsprechenden Benzimidazolons mit
Schwefelkohlenstoff.

4- bzw. 5-Methyl-2-mercaptobenzimidazol wird in der
Kautschukindustrie als Alterungsschutzmittel für
Natur- oder Synthesekautschuk alleine oder in Kombination mit weiteren üblichen Alterungsschutzmitteln
eingesetzt. Wie beispielsweise aus der deutschen
Patentschrift 557 138 hervorgeht, können die Verbindungen durch Umsetzung eines Diaminotoluols mit
Schwefelkohlenstoff erhalten werden.

Aufgabe der vorliegenden Erfindung ist es, ein
weiteres Verfahren bereitzustellen.

Die Erfindung betrifft somit eine Verfahren zur Herstellung von 4- bzw. 5-Methyl-2-mercaptobenzimidazol
das dadurch gekennzeichnet ist, daß man 1 Gew.-Teil 4-
bzw. 5-Methylbenzimidazolon mit 3 - 10 Gew.-Teilen

Le A 20 234-Ausland

Schwefelkohlenstoff in Gegenwart von 1 bis 12 Gewichtsprozent eines basischen Katalysators, bezogen auf das
Benzimidazolon, in einem Temperaturbereich von 200
bis 300°C umsetzt.

Die Umsetzung erfolgt bevorzugt in 3 bis 10 Stunden,
besonders bevorzugt in 4,5 bis 7 Stunden. Der Temperaturbereich beträgt bevorzugt 240 bis 260°C. Schwefelkohlenstoff wird bevorzugt mit 4 bis 6 Gewichtsteilen
pro 1 Gewichtsteil Methylbenzimidazolon zugegeben, der
basische Katalysator bevorzugt in Mengen von 5 bis 10
Gewichtprozent.

Bei der Umsetzung baut sich Druck auf. Es muß dafür
gesorgt werden, daß dieser Druck bestehen bleibt und
aus der benetzten Apparatur nicht entweicht.

Als basischer Katalystor wird ein Alkali- oder Erdalkali-carbonat oder -hydroxid bevorzugt verwendet,
wie beispielsweise Natrium-, Kalium-, Lithium- oder
Calciumcarbonat, oder Natrium- oder Kaliumhydroxid.

4- bzw. 5-Methylbenzimidazolon ist bekannt. Es ist
möglich, dieses Ausgangsprodukt aus einem in der Isocyanatchemie in großen Mengen anfallenden Abfallprodukt herzustellen. So beschreibt die DE-OS 2 703 313
ein Verfahren, durch das die Rückstände der Toluylendiisocyanatdestillation mittels Ammoniak in Diaminotoluol und 4- bzw. 5-Methylbenzimidazolon gespalten

Le A 20 234

werden können. Aus diesem Gemisch kann das Benzimidazolon in leichter Weise isoliert werden. Das so erhaltene Gemisch aus 4- und 5-Methylbenzimidazolon kann gemäß dem erfindungsgemäßen Verfahren in ein Gemisch aus 4- und 5-Methyl-2-mercaptobenzimidazol umgewandelt werden, welches sich hervorragend als Alterungsschutzmittel für Kautschuke eignet.

Das erfindungsgemäße Verfahren kann auf folgende Weise durchgeführt werden:

50 g 4- bzw. 5-Methylbenzimidazolon werden mit 250 g Schwefelkohlenstoff und 5 g Pottasche 5 Stunden bei 250°C gerührt. Es wird entspannt, überschüssiger Schwefelkohlenstoff entfernt, der Rückstand in verdünnter Natronlauge gelöst, die Lösung mit A-Kohle geklärt, das Filtrat mit Salzsäure angesäuert und der Niederschlag abgesaugt. Es wird mit Wasser gewaschen und im Vakuum getrocknet. Es verbleiben 51,5 g eines Produktgemisches vom Schmelzpunkt 283 - 289°C, das laut IR-Spektrum identisch ist mit einer Probe, die durch Umsetzung des 3- bzw. 4-Methyl-1,2-diaminobenzols mit Schwefelkohlenstoff gewonnen wurde.

Le A 20 234

0035185

- 4 -

Patentansprüche:

1) Verfahren zur Herstellung von 4- bzw. 5-Methyl-2-mercaptobenzimidazol, dadurch gekennzeichnet, daß man 1 Gewichtsteil 4- bzw. 5-Methylbenzimidazolon mit 3 bis 10 Gewichtsteilen Schwefelkohlenstoff in Gegenwart von 1 bis 12 Gewichtsprozent eines basischen Katalysators, bezogen auf Benzimidazolon, in einem Temperaturbereich von 200 bis 300°C umsetzt.

2) Verfahren gemäß Anspruch 1, dadurch gekennzeichnet, daß man 1 Gewichtsteil 4- bzw. 5-Methylbenzimidazolon mit 4 bis 6 Gewichtsteilen Schwefelkohlenstoff umsetzt.

3) Verfahren gemäß Anspruch 1, dadurch gekennzeichnet, daß man die Umsetzung in Gegenwart von 5 bis 10 Gewichtsprozent eines basischen Katalysators durchführt.

4) Verfahren gemäß Ansprüche 1 - 3, dadurch gekennzeichnet, daß man die Umsetzung in einem Temperaturbereich von 240 bis 260°C durchführt.

5) Verfahren nach Ansprüchen 1 - 4, dadurch gekennzeichnet, daß man als basischen Katalysator ein Alkali und/oder Erdalkalicarbonat und/oder -hydroxid einsetzt.

Le A 20 234

0035185

6) Verfahren nach Ansprüchen 1 - 5, dadurch gekennzeichnet, daß man als basischen Katalysator
Pottasche oder Kaliumhydroxid einsetzt.

7) 4- bzw. 5-Methyl-2-mercaptobenzimidazol hergestellt nach einen Verfahren der Ansprüche 1 - 6.

Le A 20 234

Europäisches
Patentamt

**EUROPÄISCHER RECHERCHENBERICHT**

0035185

Nummer der Anmeldung

EP 81101203.8

| Kategorie | Kennzeichnung des Dokuments mit Angabe, soweit erforderlich, der maßgeblichen Teile | betrifft Anspruch |
|---|---|---|
| | DE - A - 2 415 312 (DU PONT) <br> + Seite 14 + <br> -- | 1 |
| A | DE - A - 2 202 204 (BAYER) <br> + Seite 9 + <br> ---- | 1 |

**EINSCHLÄGIGE DOKUMENTE**

**KLASSIFIKATION DER ANMELDUNG (Int. Cl.³)**

C 07 D 235/28

**RECHERCHIERTE SACHGEBIETE (Int. Cl.³)**

C 07 D 235/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 20-05-1981 | BRUS |

EPA form 1503.1 06.78